(19) Europäisches Patentamt
European Patent Office
Office européen des brevets

(11) **EP 4 137 036 A1**

(12) **EUROPEAN PATENT APPLICATION**

(43) Date of publication:
**22.02.2023 Bulletin 2023/08**

(21) Application number: **22189664.0**

(22) Date of filing: **10.08.2022**

(51) International Patent Classification (IPC):
**A61B 3/06** (2006.01)

(52) Cooperative Patent Classification (CPC):
**A61B 3/066**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.08.2021 JP 2021132593**

(71) Applicant: **Chukyo Medical Co., Inc.**
**Nagoya-shi, Aichi 456-0032 (JP)**

(72) Inventors:
• **ICHIKAWA, Kazuo**
 **Aichi (JP)**
• **YOKOYAMA, Sho**
 **Aichi (JP)**
• **TANAKA, Yoshiki**
 **Aichi (JP)**

(74) Representative: **Ter Meer Steinmeister & Partner**
**Patentanwälte mbB**
**Nymphenburger Straße 4**
**80335 München (DE)**

(54) **VISUAL TARGET DISPLAY DEVICE, VISUAL TARGET PRESENTATION METHOD, AND PROGRAM**

(57)    Provided is a visual target display device or a visual target presentation method which can test the cone-specific visual function more accurately in a device that displays a visual target, of a chromatic color, for testing the color-related visual function of a subject or a method of presenting the visual target.

A visual target 8, of a chromatic color, for testing the color-related visual function of a subject and a background 9 around the visual target 8 are presented in such a way that the brightness of the background 9 is a numerical value between a numerical value 10% less than the brightness of the visual target 8 and a numerical value 10% greater than the brightness of the visual target 8 and a combination of the color of the visual target 8 and the color of the background 9 is a combination of colors which are in a relationship of confusion colors in dichromatism. The color of the background 9 is set to a chromatic color, for example. For example, the color of the visual target 8 and the color of the background 9 are set to chromatic colors located on opposite sides of a region of an achromatic color on the same confusion color locus passing through the region of the achromatic color in a chromaticity diagram. The color of the visual target 8 and the color of the background 9 are set to colors of the same chroma, for example.

FIG.1

EP 4 137 036 A1

**Description**

BACKGROUND OF THE INVENTION

Field of the Invention

This disclosure relates to a device that displays a visual target, of a chromatic color, for testing the color-related visual function of a subject and a method of presenting the visual target.

Description of Related Art

A large number of cone cells (L-cones, M-cones, and S-cones) that perceive three colors, i.e., red, green, and blue, are arranged in a retina, which provides the human visual function with the ability to perceive various colors. An abnormality in a cone cell can cause an abnormality in the ability to perceive color. Japanese Patent Nos. 5436712 and 6184046 describe presenting a visual target of a chromatic color to a subject in order to test the ability to perceive color. Moreover, Japanese Patent Nos. 5436712 and 6184046 propose making a visual target and a background have similar brightnesses (a brightness difference of ±10%) in order to properly test perception of a difference in color, not perception of a difference in brightness.

SUMMARY OF THE INVENTION

In testing the color-related visual function (the ability to see color), it is desirable to be able to make more accurate determination about the presence or absence of color vision deficiency (an abnormality in the ability to perceive color) and the degree of color vision deficiency. This makes it desirable to be able to test the cone-specific visual function (the presence or absence of an abnormality and the degree of an abnormality), that is, the visual function of L-cones, the visual function of M-cones, and the visual function of S-cones more accurately. In other words, it is desirable that the test results indicate, on a cone-by-cone basis, the presence or absence of an abnormality, the degree of an abnormality, or the sensitivity of a cone.

Therefore, an object of this disclosure is to provide a visual target display device that can test the cone-specific visual function more accurately, a visual target presentation method that can test the cone-specific visual function more accurately, or a program that can test the cone-specific visual function more accurately.

In order to solve the above-mentioned problem, a first aspect of a visual target display device of this disclosure includes:

a display section configured to display a visual target, of a chromatic color, for testing a color-related visual function of a subject and a background around the visual target, wherein

a brightness of the background is a numerical value between a numerical value 10% less than a brightness of the visual target and a numerical value 10% greater than the brightness of the visual target,

a combination of a color of the visual target and a color of the background is a combination of colors which are in a relationship of confusion colors in dichromatism, and

the display section changes a size of the visual target or displays a plurality of the visual targets of different sizes while maintaining the combination of the color of the visual target and the color of the background, so as to obtain a threshold value which is a size of the visual target based on which a determination on whether or not the subject is able to perceive the visual target is made.

A second aspect of the visual target display device of this disclosure includes:

a display section configured to display a visual target, of a chromatic color, for testing a color-related visual function of a subject and a background around the visual target, wherein

a brightness of the background is a numerical value between a numerical value 10% less than a brightness of the visual target and a numerical value 10% greater than the brightness of the visual target,

a combination of a color of the visual target and a color of the background is a combination of colors which are in a relationship of confusion colors in dichromatism,

the visual target has a shape in which a plurality of minimum constituent units set to a same shape and a same color are arranged in columns and rows, and

the visual target is a visual target for testing whether or not there is a region which the subject is not able to see in the visual target or a region which looks distorted in the visual target while the subject is looking at a center of the visual target.

Moreover, a visual target presentation method of this disclosure includes:

presenting a visual target, of a chromatic color, for testing a color-related visual function of a subject and a background around the visual target in such a way that a brightness of the background is a numerical value between a numerical value 10% less than a brightness of the visual target and a numerical value 10% greater than the brightness of the visual target and a combination of a color of the visual target and a color of the background is a combination of colors which are in a relationship of confusion colors in dichromatism; and

obtaining a threshold value, which is a size of the visual target based on which a determination on whether or not the subject is able to perceive the visual target is made, by changing a size of the visual target presented to the subject while maintaining the combination of the color of the visual target and the color of the background.

Furthermore, a program of this disclosure for causing a computer to function as display control means configured to cause a display section to display a visual target, of a chromatic color, for testing a color-related visual function of a subject and a background around the visual target in such a way that a brightness of the background is a numerical value between a numerical value 10% less than a brightness of the visual target and a numerical value 10% greater than the brightness of the visual target and a combination of a color of the visual target and a color of the background is a combination of colors which are in a relationship of confusion colors in dichromatism, wherein

the display control means changes a size of the visual target while maintaining the combination of the color of the visual target and the color of the background, so as to obtain a threshold value which is a size of the visual target based on which a determination on whether or not the subject is able to perceive the visual target is made.

The visual target display device, the visual target presentation method, or the program of this disclosure makes it possible to properly test perception of a visual target (chromatic color), not perception based on a brightness difference between a background and a visual target, because a background and a visual target have similar brightnesses (a brightness difference falls within ±10%). Moreover, a visual target color and a background color are in a relationship of confusion colors in dichromatism. Dichromatism refers to a condition in which the sensitivity of any one of an L-cone, an M-cone, and an S-cone is extremely low, and includes protanopia which is a condition in which the sensitivity of L-cones is low, deuteranopia which is a condition in which the sensitivity of M-cones is low, and tritanopia which is a condition in which the sensitivity of S-cones is low. Furthermore, confusion colors in dichromatism refer to a combination of colors whose difference is not discernible by a dichromat. For example, when a visual target color and a background color are in a relationship of confusion colors in protanopia, the lower the sensitivity of L-cones, the more difficult perception of the visual target becomes. In this case, a difference in test results is more likely to be apparent between a person with an abnormality in L-cones and a person without an abnormality in L-cones or between a person with L-cones having high sensitivity and a person with L-cones having low sensitivity, which makes it possible to test the visual function related to L-cones more accurately. When a visual target color and a background color are in a relationship of confusion colors in deuteranopia, it is possible to test the visual function related to M-cones more accurately. When a visual target color and a background color are in a relationship of confusion colors in tritanopia, it is possible to test the visual function related to S-cones more accurately. The above description basically deals with characteristics in congenital color vision deficiency. However, similar characteristics apply to acquired color vision deficiency because various eye diseases can cause a cone disorder. As described above, in this disclosure, it is possible to test the cone-specific visual function more accurately.

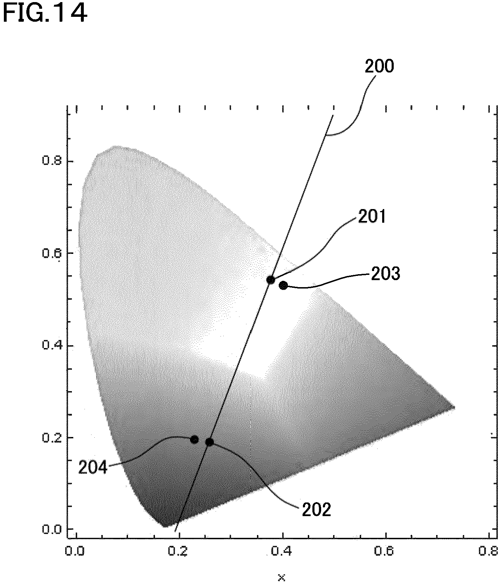

It is assumed that, in a chromaticity diagram, a line passing through coordinates (chromaticity values) in a relationship of confusion colors is a confusion color locus, a color that is located exactly on the confusion color locus is an on-locus color, and a color whose color difference ΔE from the on-locus color in the CIE1976L*a*b* color space is 10.0 or less is a similar color. Cases where "the color of a visual target and the color of a background are in a relationship of confusion colors in dichromatism" in this disclosure include a case where a visual target color and a background color are different on-locus colors on the same confusion color locus, a case where one of a visual target color and a background color is one on-locus color and the other is a color (similar color) similar to another on-locus color on a confusion color locus passing through the one on-locus color, and a case where a visual target color and a background color are colors similar to different on-locus colors on the same confusion color locus. These cases will be explained taking FIG. 14 as an example. In FIG. 14, it is assumed that points 201 and 202 are different on-locus colors on the same confusion color locus 200, a point 203 is a color similar to the on-locus color 201, and a point 204 is a color similar to the on-locus color 202. In this example, a case where one of a visual target color and a background color is the on-locus color 201 and the other is the on-locus color 202 corresponds to the above-described "case where a visual target color and a background color are different on-locus colors on the same confusion color locus". Moreover, a case where one of a visual target color and a background color is the on-locus color 201 and the other is the similar color 204 or a case where one of a visual target color and a background color is the on-locus color 202 and the other is the similar color 203 corresponds to the above-described "case where one of a visual target color and a background color is one on-locus color and the other is a color similar to another on-locus color on a confusion color locus passing through the one on-locus color".

Furthermore, a case where one of a visual target color and a background color is the similar color 203 and the other is the similar color 204 corresponds to the above-described "case where a visual target color and a background color are colors similar to different on-locus colors on the same confusion color locus".

Therefore, it can be said that a visual target color and a background color are in a relationship of confusion colors in dichromatism in both cases where the background color is an on-locus color that is located on a confusion color locus passing through the visual target color and is different from the visual target color and where the background color is a color similar to the on-locus color. Moreover, it can be said that a visual target color and a background color are in a relationship of confusion colors in dichromatism in both cases where the visual target color is an on-locus color that is located on a confusion color locus passing through the background color and is different from the background color and where the visual target color is a color similar to the on-locus color. It is to be noted that the CIE1976L*a*b* color space is a uniform color space defined by the International Commission on Illumination (CIE) in 1976.

Moreover, the "visual target display device" and the "display section" in this disclosure include various display means that can display a visual target, such as sheets including paper, resin, or the like on which a visual target is displayed (for example, printed) and projection equipment (a projector) that projects a visual target, in addition to displays that electrically display an image, such as a liquid crystal display, a plasma display, and an organic EL display.

Furthermore, an "achromatic color" in this disclosure is a color near the center ((x, y)=(0.33, 0.33)) of a chromaticity diagram (an xy chromaticity diagram) in the CIE1931XYZ color space (a color space defined by the CIE in 1931), and specifically, it refers to a color whose chromaticity values x and y are each in the range of 0.30 to 0.350, for example. Moreover, a "chromatic color" refers to a color other than the achromatic color.

In addition, the brightness of a color refers to a Y value of the tristimulus values X, Y, and Z.

BRIEF DESCRIPTION OF THE DRAWINGS

FIG. 1 is a block diagram showing the configuration of a visual target display device;
FIG. 2 is a diagram showing the configuration of visual target data;
FIG. 3 is a diagram showing, on an xy chromaticity diagram, confusion color loci of protanopia, deuteranopia, and tritanopia and chroma-by-chroma chromaticity points of 15 colors which are used in NEW COLOR TEST;
FIG. 4 is a diagram illustrating a plurality of confusion color loci of protanopia on an xy chromaticity diagram;
FIG. 5 is a diagram illustrating a plurality of confusion color loci of deuteranopia on an xy chromaticity diagram;
FIG. 6 is a diagram illustrating a plurality of confusion color loci of tritanopia on an xy chromaticity diagram;
FIG. 7 is a flowchart showing a procedure for testing the ability to see color of a first embodiment;
FIG. 8 is a diagram illustrating display on a display section of a second embodiment;
FIG. 9 is a flowchart showing a procedure for testing the ability to see color of the second embodiment;
FIG. 10 is a diagram illustrating display on a display section of a third embodiment;
FIG. 11 is a flowchart showing a procedure for testing the ability to see color of the third embodiment;
FIG. 12 is a diagram illustrating a sheet on which Landolt rings are printed as visual targets;
FIG. 13 is a diagram illustrating a sheet on which a grid is printed as a visual target; and
FIG. 14 is a diagram illustrating, on an xy chromaticity diagram, a confusion color locus, on-locus colors located on the confusion color locus, and similar colors similar to the on-locus colors.

DESCRIPTION OF THE PREFERRED EMBODIMENTS

First Embodiment

Hereinafter, a first embodiment of this disclosure will be described with reference to the drawings. In this disclosure, color-specific visual acuity (for example, the ability to identify a red object with eyes and the ability to identify a blue object with eyes) is referred to as "color visual acuity", the color-specific visual field (for example, the range of peripheral vision in which a red color is able to be perceived without the movement of eyes and the range of peripheral vision in which a blue color is able to be perceived without the movement of eyes) is referred to as the "visual field of color", and the color visual acuity and the visual field of color are collectively referred to as the "ability to see color".

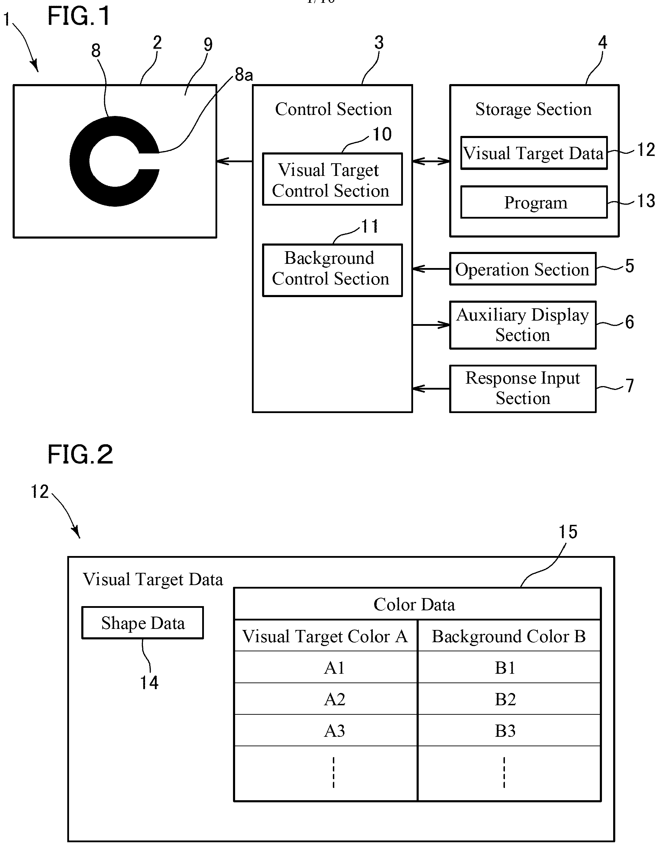

The first embodiment is an embodiment in which color visual acuity is mainly tested, specifically, an embodiment in which a chromatic Landolt ring is presented in the center of the visual field of a subject as a visual target to test whether or not the subject is able to perceive the Landolt ring. FIG. 1 shows the configuration of a visual target display device of this embodiment. A visual target display device 1 of FIG. 1 includes a display section 2, a control section 3, a storage section 4, an operation section 5, an auxiliary display section 6, and a response input section 7. The response input section 7 is used in a second embodiment, which will be described later, and is not used in the first embodiment.

Therefore, the visual target display device 1 of the first embodiment does not necessarily have to include the response input section 7.

The display section 2 is a section that displays various images, and is configured to be able to change the shape or color (including a background color) of a displayed image (visual target) to another shape or color. The display section 2 is a display that displays an image using light emission generated by supply of power, and is a liquid crystal display, for example. The display section 2 may be other types of display such as a plasma display and an organic EL display. Moreover, the display section 2 may be a head-mounted display.

The display section 2 displays a visual target 8 and a background 9 around the visual target 8. In this embodiment, the display section 2 displays one visual target 8 at a time. The visual target 8 is a Landolt ring. The Landolt ring 8 is a ring-shaped image which is cut in a part thereof. The subject is asked which direction a gap 8a of the Landolt ring 8 faces. The background 9 is a plain image (without any pattern) of a single color.

The visual target 8 and the background 9 are set to virtually the same brightness. Specifically, the brightness of the background 9 is set to a numerical value between a numerical value 10% less than the brightness of the visual target 8 and a numerical value 10% greater than the brightness of the visual target 8. The brightness of the visual target 8 and the brightness of the background 9 are set to 30 cd/m$^2$, for example.

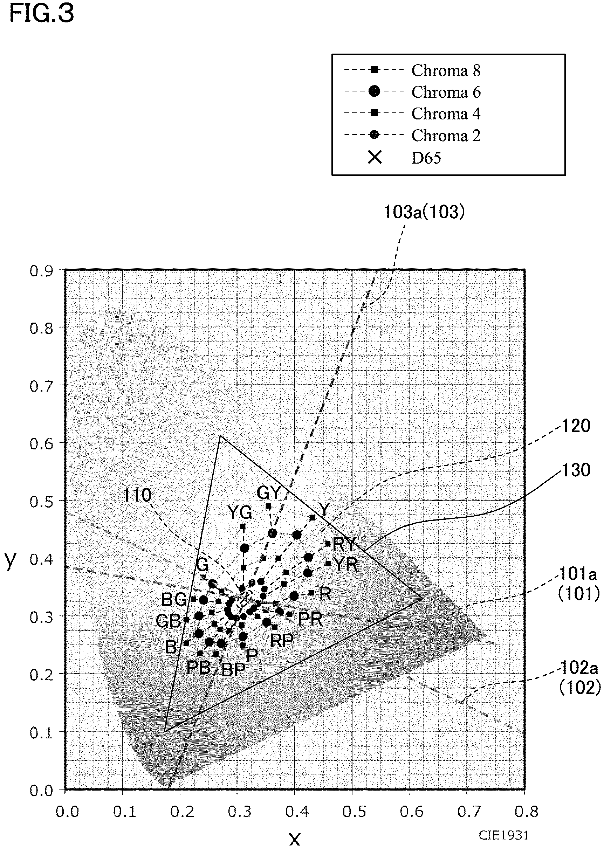

Moreover, the color of the visual target 8 and the color of the background 9 are set to a combination of colors which are in a relationship of confusion colors in dichromatism. Specifically, FIG. 3 illustrates, in a CIE1931 xy chromaticity diagram, a confusion color locus 101 in protanopia (hereinafter sometimes referred to as a protanopia confusion color locus), a confusion color locus 102 in deuteranopia (hereinafter sometimes referred to as a deuteranopia confusion color locus), and a confusion color locus 103 in tritanopia (hereinafter sometimes referred to as a tritanopia confusion color locus).

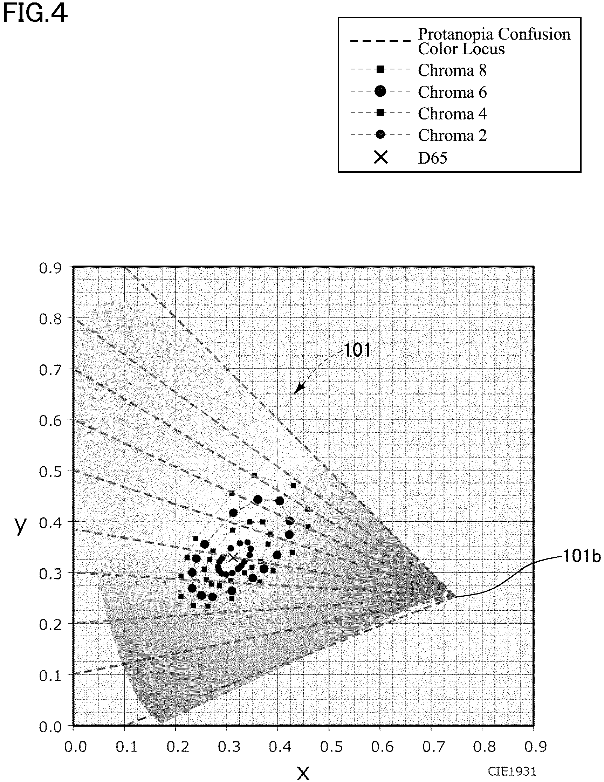

As shown in FIG. 4, the protanopia confusion color locus 101 is a straight line passing through a particular point 101b (which is referred to as a confusion color center) in an xy chromaticity diagram, and there are an infinite number of protanopia confusion color loci 101. The chromaticity values (x, y) of the confusion color center 101b of the protanopia confusion color loci 101 are (0.747, 0.253). In colors on the same confusion color locus 101, the amount of response of M-cones and the amount of response of S-cones do not change and only the amount of response of L-cones changes. FIG. 3 illustrates one locus 101a of an infinite number of protanopia confusion color loci 101, the locus 101a passing through an achromatic region 110 of the chromaticity diagram. Specifically, this confusion color locus 101a is a straight line passing through a point of D65 illuminant color in the achromatic region 110. The chromaticity values (x, y) of D65 for the 2° standard colorimetric observer in the xy chromaticity diagram are (0.3127, 0.3290).

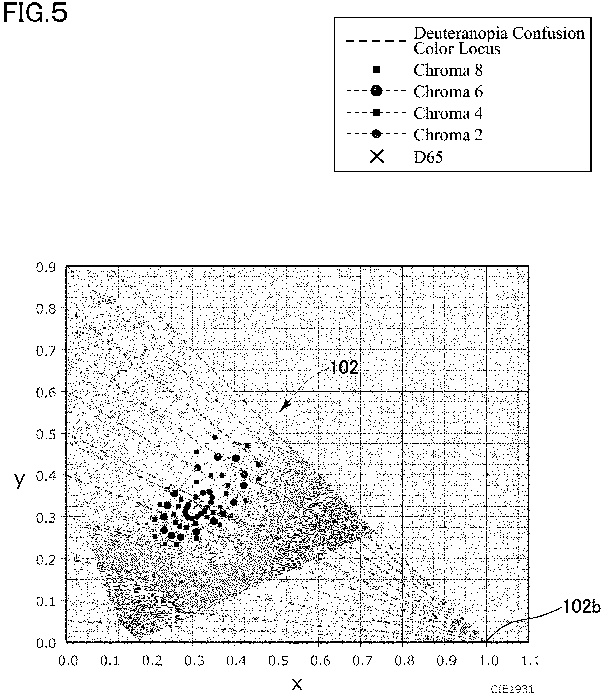

Furthermore, as shown in FIG. 5, the deuteranopia confusion color locus 102 is a straight line passing through a particular point 102b (which is referred to as a confusion color center) in an xy chromaticity diagram, and there are an infinite number of deuteranopia confusion color loci 102. The chromaticity values (x, y) of the confusion color center 102b of the deuteranopia confusion color loci 102 are (1.000, 0.000). In colors on the same confusion color locus 102, the amount of response of L-cones and the amount of response of S-cones do not change and only the amount of response of M-cones changes. FIG. 3 illustrates a locus 102a of an infinite number of deuteranopia confusion color loci 102, the locus 102a passing through the point of D65.

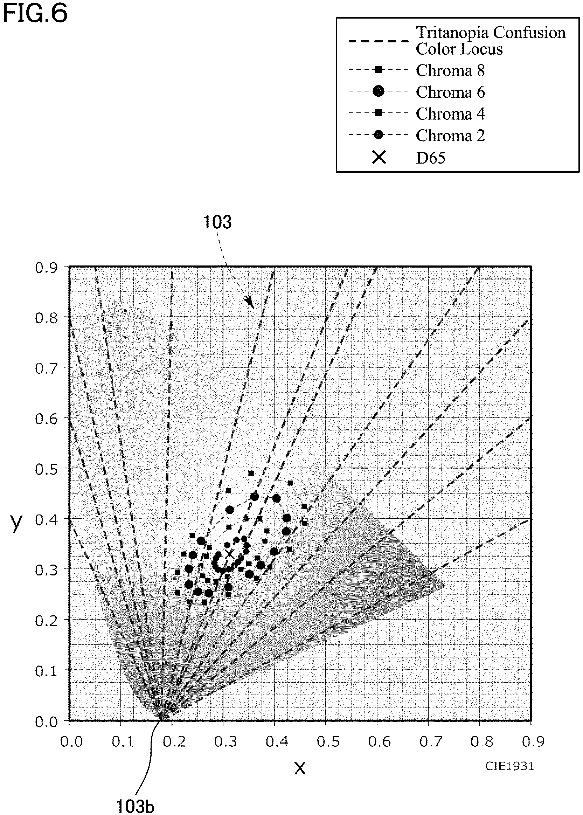

In addition, as shown in FIG. 6, the tritanopia confusion color locus 103 is a straight line passing through a particular point 103b (which is referred to as a confusion color center) in an xy chromaticity diagram, and there are an infinite number of tritanopia confusion color loci 103. The chromaticity values (x, y) of the confusion color center 103b of the tritanopia confusion color loci 103 are (0.180, 0.000). In colors on the same confusion color locus 103, the amount of response of L-cones and the amount of response of M-cones do not change and only the amount of response of S-cones changes. FIG. 3 illustrates a locus 103a of an infinite number of tritanopia confusion color loci 103, the locus 103a passing through the point of D65.

The color of the visual target 8 and the color of the background 9 may be set to a combination of colors which are in a relationship of confusion colors in protanopia, for example. In this case, the color of the visual target 8 and the color of the background 9 may be set to different chromatic colors (on-locus colors) on the protanopia confusion color locus 101a of FIG. 3. Alternatively, one of the color of the visual target 8 and the color of the background 9 may be set to a chromatic color (an on-locus color) on the protanopia confusion color locus 101a and the other may be set to a color similar to a color on the protanopia confusion color locus 101a. Alternatively, the color of the visual target 8 may be set to a color similar to an on-locus color on the protanopia confusion color locus 101a and the color of the background 9 may be set to a color similar to another on-locus color on the protanopia confusion color locus 101a.

Moreover, the color of the visual target 8 and the color of the background 9 may be set to chromatic colors (opposite colors) (on-locus colors or colors similar to them) located on opposite sides of the achromatic region 110 (the point of D65) on the protanopia confusion color locus 101a, for example. In this case, for example, the color of the visual target 8 and the color of the background 9 may be set to opposite colors (on-locus colors or colors similar to them) of the same chroma (for example, chroma 6) on the protanopia confusion color locus 101a or may be set to opposite colors (on-locus colors or colors similar to them) of different chromas on the protanopia confusion color locus 101a. Alternatively, the color of the visual target 8 and the color of the background 9 may be set to a combination of colors of different chromas in the same region out of two chromatic regions on the protanopia confusion color locus 101a which are sectioned with the achromatic region 110 (the point of D65) located therebetween.

FIG. 3 shows the chromaticity points of 15 colors of chromas 2, 4, 6, 8 which are used in NEW COLOR TEST. Specifically, the 15 colors are red (R), yellow-red (YR), red-yellow (RY), yellow (Y), green-yellow (GY), yellow-green (YG), green (G), blue-green (BG), green-blue (GB), blue (B), purple-blue (PB), blue-purple (BP), purple (P), red-purple (RP), and purple-red (PR). The above-described chromas 2, 4, 6, 8 are values in the Munsell color system. Moreover, FIG. 3 shows, for each chroma, a ring-shaped line 120 connecting the chromaticity points of the 15 colors. Of the 15 colors, a combination of opposite colors located near the protanopia confusion color locus 101a is a combination of blue-green (BG) and purple-red (PR). Therefore, a combination of colors of the visual target 8 and the background 9 may be set to a combination of blue-green (BG) and purple-red (PR), for example. It is to be noted that NEW COLOR TEST is a test to determine the presence or absence of color vision deficiency by getting the subject to rearrange the randomly-arranged 15 colors mentioned above in the order in which they gradually change in hue.

Even when the chromaticity point of blue-green (BG) or the chromaticity point of purple-red (PR) which is used in NEW COLOR TEST does not correspond to an on-locus color that is located exactly on the protanopia confusion color locus 101a, the color of the chromaticity point which is used in NEW COLOR TEST may be set as the color of the visual target 8 or the background 9 as it is when a color difference $\Delta E$ from the on-locus color in the CIE1976L*a*b* color space is 10.0 or less. Moreover, when the above-mentioned color difference $\Delta E$ is more than 10.0, the color of the chromaticity point which is used in NEW COLOR TEST may be corrected in such a way that the color difference $\Delta E$ is 10.0 or less and the corrected color may be set as the color of the visual target 8 or the background 9. Furthermore, for example, when the visual target 8 and the background 9 are set to colors of chroma 6, if the above-mentioned color difference $\Delta E$ (a color difference between a chromaticity point that is located exactly on the locus 101a and a chromaticity point which is used in NEW COLOR TEST) is more than 10.0, the above-described correction may be made in such a way that the corrected color is a point on the ring-shaped line 120 representing chroma 6 in FIG. 3.

The color of the visual target 8 and the color of the background 9 may be set to a combination of colors which are in a relationship of confusion colors in deuteranopia, for example. In this case, the color of the visual target 8 and the color of the background 9 may be set to different chromatic colors (on-locus colors or colors similar to them) on the deuteranopia confusion color locus 102a of FIG. 3. Alternatively, the color of the visual target 8 and the color of the background 9 may be set to chromatic colors (opposite colors) (on-locus colors or colors similar to them) located on opposite sides of the achromatic region 110 (the point of D65) on the deuteranopia confusion color locus 102a, for example. In this case, for example, the color of the visual target 8 and the color of the background 9 may be set to opposite colors (on-locus colors or colors similar to them) of the same chroma (for example, chroma 6) on the deuteranopia confusion color locus 102a or may be set to opposite colors (on-locus colors or colors similar to them) of different chromas on the deuteranopia confusion color locus 102a. Alternatively, the color of the visual target 8 and the color of the background 9 may be set to a combination of colors of different chromas in the same region out of two chromatic regions on the deuteranopia confusion color locus 102a which are sectioned with the achromatic region 110 (the point of D65) located therebetween.

Of the above-mentioned 15 colors which are used in NEW COLOR TEST, a combination of opposite colors located near the deuteranopia confusion color locus 102a is a combination of green (G) and purple-red (PR). Therefore, a combination of colors of the visual target 8 and the background 9 may be set to a combination of green (G) and purple-red (PR), for example. In this case, even when the chromaticity point of green (G) or the chromaticity point of purple-red (PR) does not correspond to an on-locus color that is located exactly on the deuteranopia confusion color locus 102a, the color of this chromaticity point may be set as the color of the visual target 8 or the background 9 as it is when a color difference $\Delta E$ from the on-locus color in the CIE1976L*a*b* color space is 10.0 or less. Moreover, when the above-mentioned color difference $\Delta E$ is more than 10.0, the chromaticity point of green (G) or purple-red (PR) may be corrected in such a way that the color difference $\Delta E$ is 10.0 or less and the corrected color may be set as the color of the visual target 8 or the background 9. In this case, the above-described correction may be made in such a way that the chromaticity point after the correction is a point on the ring-shaped line 120 (see FIG. 3) representing the chroma of the chromaticity point before the correction.

The color of the visual target 8 and the color of the background 9 may be set to a combination of colors which are in a relationship of confusion colors in tritanopia, for example. In this case, the color of the visual target 8 and the color of the background 9 may be set to different chromatic colors (on-locus colors or colors similar to them) on the tritanopia confusion color locus 103a of FIG. 3. Alternatively, the color of the visual target 8 and the color of the background 9 may be set to chromatic colors (opposite colors) (on-locus colors or colors similar to them) located on opposite sides of the achromatic region 110 (the point of D65) on the tritanopia confusion color locus 103a, for example. In this case, for example, the color of the visual target 8 and the color of the background 9 may be set to opposite colors (on-locus colors or colors similar to them) of the same chroma (for example, chroma 6) on the tritanopia confusion color locus 103a or may be set to opposite colors (on-locus colors or colors similar to them) of different chromas on the tritanopia confusion color locus 103a. Alternatively, the color of the visual target 8 and the color of the background 9 may be set to a combination of colors of different chromas in the same region out of two chromatic regions on the tritanopia confusion color locus 103a which are sectioned with the achromatic region 110 (the point of D65) located therebetween.

Of the above-mentioned 15 colors which are used in NEW COLOR TEST, a combination of opposite colors located near the tritanopia confusion color locus 103a is a combination of green-yellow (GY) and blue-purple (BP). Therefore, a combination of colors of the visual target 8 and the background 9 may be set to a combination of green-yellow (GY) and blue-purple (BP), for example. In this case, even when the chromaticity point of green-yellow (GY) or the chromaticity point of the blue-purple (BP) does not correspond to an on-locus color that is located exactly on the tritanopia confusion color locus 103a, the color of this chromaticity point may be set as the color of the visual target 8 or the background 9 as it is when a color difference $\Delta E$ from the on-locus color in the CIE1976L*a*b* color space is 10.0 or less. Moreover, when the above-mentioned color difference $\Delta E$ is more than 10.0, the chromaticity point of green-yellow (GY) or blue-purple (BP) may be corrected in such a way that the color difference $\Delta E$ is 10.0 or less and the corrected color may be set as the color of the visual target 8 or the background 9. In this case, the above-described correction may be made in such a way that the chromaticity point after the correction is a point on the ring-shaped line 120 (see FIG. 3) representing the chroma of the chromaticity point before the correction.

When the three-dimensional coordinates of a color P in the CIE1976L*a*b* color space are assumed to be $(L^*_P, a^*_P, b^*_P)$ and the three-dimensional coordinates of a color Q in the CIE1976L*a*b* color space are assumed to be $(L^*_Q, a^*_Q, b^*_Q)$, a color difference $\Delta E$ between the color P and the color Q is expressed by the following formula.

[Math. 1]

$$\Delta E = \sqrt{(\Delta L^*)^2 + (\Delta a^*)^2 + (\Delta b^*)^2}$$

$$(\text{where} \quad \Delta L^* = L^*_Q - L^*_P \, , \, \Delta a^* = a^*_Q - a^*_P \, , \, \Delta b^* = b^*_Q - b^*_P)$$

Moreover, conversion from the tristimulus values (X, Y, Z) in the CIE1931XYZ color space to the coordinates (L*, a*, b*) in the CIEI976L*a*b* color space is expressed by the following formula. It is to be noted that $X_n, Y_n, Z_n$ in the following formula are tristimulus values relative to the perfect reflecting diffuser (standard white surface). If D65 is a standard white surface, then $X_n = 95.047$, $Y_n = 100.00$, and $Z_n = 108.883$.

[Math. 2]

$$L^* = 116 f(Y/Y_n) - 16$$

$$a^* = 500[f(X/X_n) - f(Y/Y_n)]$$

$$b^* = 200[f(Y/Y_n) - f(Z/Z_n)]$$

where

$$f(t) = \begin{cases} t^{1/3} & t > (6/29)^3 \\ \frac{1}{3}\left(\frac{29}{6}\right)^2 t + \frac{4}{29} & otherwise \end{cases}$$

Furthermore, when the visual target color or the background color is set to a similar color (a color whose color difference $\Delta E$ from an on-locus color is 10.0 or less) which is not exactly the same as a color (an on-locus color) on a given confusion color locus, it is preferable that a color difference $\Delta E$ between the visual target color or the background color and the on-locus color is small from the viewpoint of testing the ability to see color more accurately. Specifically, the visual target color or the background color may be set to a similar color whose $\Delta E$ is 5.0 or less, for example, preferably to a similar color whose $\Delta E$ is 3.0 or less, more preferably to a similar color whose $\Delta E$ is 1.0 or less, further preferably to a similar color whose $\Delta E$ is 0.5 or less, still further preferably to a similar color whose $\Delta E$ is 0.3 or less, and even more preferably to a similar color whose $\Delta E$ is 0.1 or less.

In addition, the color of the visual target 8 and the color of the background 9 are set to colors which can be reproduced by the display section 2. Specifically, the color of the visual target 8 and the color of the background 9 are set to colors in the sRGB (standard RGB) color gamut 130 shown in FIG. 3, for example. It is to be noted that sRGB is the international standard of color space established by the International Electrotechnical Commission (IEC).

Back in FIG. 1, the control section 3 is configured with a CPU, a ROM, a RAM, and the like and controls display on the display section 2 and the auxiliary display section 6 which will be described later. Specifically, the control section 3 includes a visual target control section 10 that controls the color (including brightness), size, and orientation of the visual target 8 which is displayed by the display section 2 and a background control section 11 that controls the color (including brightness) of the background 9 which is displayed by the display section 2. As described earlier, the visual target control section 10 and the background control section 11 control the brightness of the visual target 8 and the brightness of the background 9 in such a way that a brightness difference between the visual target 8 and the background 9 falls within plus or minus 10% of the brightness of the visual target 8 or the brightness of the background 9 (that is, in such a way that the visual target 8 and the background 9 have virtually the same brightness). Moreover, as described earlier, the visual target control section 10 and the background control section 11 control the color of the visual target 8 and the color of the background 9 in such a way that a combination thereof is a combination of colors which are in a relationship of confusion colors in dichromatism.

The storage section 4 is a non-transitory tangible storage medium having stored therein a computer-readable program and data in a non-transitory manner. The non-transitory tangible storage medium is implemented by a semiconductor memory, a magnetic disk, or the like. Visual target data 12 and a program 13 of processing which is executed by the control section 3 are stored in the storage section 4. The storage section 4 is electrically connected to the control section 3. The storage section 4 may be configured as a built-in storage section which is built into the control section 3 or may be configured as an external storage section attached to the control section 3.

As shown in FIG. 2, the visual target data 12 includes shape data 14 indicating the shape of the visual target 8 and color data 15 indicating the color (including brightness) of a visual target and the color (including brightness) of a background. The shape data 14 is composed of data indicating the shapes (each being the shape of a ring which is cut in a part thereof) of a plurality of Landolt rings of different sizes and orientations.

The color data 15 includes a plurality of pieces of color data A1, A2, A3, ... indicating the color of the visual target 8 and a plurality of pieces of color data B1, B2, B3, ... indicating the color of the background 9. Each of the plurality of pieces of visual target color data A1, A2, A3, ... and each of the plurality of pieces of background color data B1, B2, B3, ... are correlated with each other on a one-to-one basis, and visual target color data A (A1, A2, A3, ...) and background color data B (B1, B2, B3, ...) correlated with each other on a one-to-one basis are set to a combination of colors which have virtually the same brightness and are in a relationship of confusion colors in dichromatism. For example, a first combination (A1, B1) of the visual target color data A and the background color data B is set to a combination of colors (for example, blue-green (BG) and purple-red (PR)) which are in a relationship of confusion colors in protanopia. Moreover, for example, a second combination (A2, B2) of the visual target color data A and the background color data B is set to a combination of colors (for example, green (G) and purple-red (PR)) which are in a relationship of confusion colors in deuteranopia. Furthermore, for example, a third combination (A3, B3) of the visual target color data A and the background color data B is set to a combination of colors (for example, green-yellow (GY) and blue-purple (BP)) which are in a relationship of confusion colors in tritanopia.

The visual target color data A and the background color data B may be color data obtained by measuring the chromaticity values (tristimulus values X, Y, and Z) of a color (including brightness), which is output from the display section 2, by a chromoscope and adjusting the measurement values in advance so that the values are desired values. Moreover, the visual target color data A and the background color data B may be data conforming to the display system of the display section 2, specifically, RGB (that is, data indicating the color mixture amounts of R (red), G (green), and B (blue)).

Back in FIG. 1, the operation section 5 is a section on which an input operation is performed by an examiner (such as a doctor), and is a keyboard, a mouse, a touch panel, or the like, for example. Specifically, the operation section 5 is a section to which a signal for specifying the shape (size, orientation) of the visual target 8 which is displayed by the display section 2, the visual target color, and the background color is input. The operation section 5 is electrically connected to the control section 3.

The auxiliary display section 6 is a section that displays information for an examiner (such as a doctor), and is a liquid crystal display, for example. The auxiliary display section 6 displays a screen for specifying the shape (size, orientation) of the visual target 8 which is displayed by the display section 2, the visual target color, and the background color. The auxiliary display section 6 is electrically connected to the control section 3.

The response input section 7 is explained in the second embodiment which will be described later.

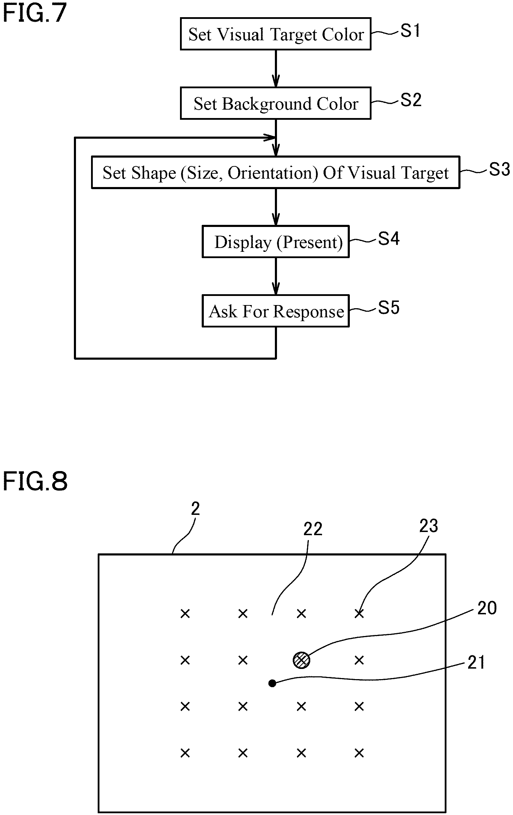

Next, a procedure of a method of testing color visual acuity using the visual target display device 1 will be described with reference to FIG. 7. A test is carried out in a semi-dark room or a dark room to eliminate the influence of extraneous light as much as possible. The subject is positioned at a predetermined distance from a screen of the display section 2. The examiner starts the program 13 (see FIG. 1) and causes the control section 3 to execute the program 13. When the program 13 is started, the control section 3 causes the auxiliary display section 6 to display the screen for specifying the shape (size, orientation) of the visual target 8, the visual target color, and the background color. For example, combinations (A1, B1), (A2, B2), (A3, B3), ... of the visual target color and the background color indicated by the color data 15 of FIG. 2 are displayed on this screen.

The examiner specifies the visual target color and the background color by operating the operation section 5 while viewing the screen of the auxiliary display section 6. The control section 3 sets the visual target color and the background color based on an input signal from the operation section 5 (Steps S1 and S2 of FIG. 7). For example, when the input signal from the operation section 5 is a signal indicating the first combination (A1, B1), the control section 3 sets the color represented by A1 as the visual target color (S1) and sets the color represented by B1 as the background color (S2).

Moreover, the examiner specifies the shape (size, orientation) of the visual target 8 by operating the operation section 5 while viewing the screen of the auxiliary display section 6. The control section 3 sets the shape of the visual target 8 based on an input signal from the operation section 5 (Step S3 of FIG. 7).

Then, the control section 3 presents the visual target 8 to the subject by causing the display section 2 to display the visual target 8 and the background 9 of the colors and shape set in Steps S1 to S3 (Step S4 of FIG. 7). Specifically, in Step S4, the visual target control section 10 (see FIG. 1) of the control section 3 reads the visual target color data A (see FIG. 2) corresponding to the color set in Step S1 and the shape data 14 (see FIG. 2) corresponding to the shape set in Step S3 from the storage section 4 and causes the display section 2 to display the visual target 8 indicated by the read visual target color data A and shape data 14. Moreover, the background control section 11 (see FIG. 1) of the control section 3 reads the background color data B (see FIG. 2) corresponding to the color set in Step S2 from the storage section 4 and causes the display section 2 to display the background 9 indicated by the read background color data B.

The examiner asks the subject to make a verbal response as to which orientation of the visual target 8 (the gap 8a) displayed on the display section 2 (Step S5 of FIG. 7) is directed.

The visual target 8 of another shape (size, orientation) is presented to the subject by repeating Steps S3 to S5 with the combination of the visual target color and the background color being maintained at the combination set in Steps S1 and S2. Finally, a threshold value, which is the size of the visual target 8 based on which a determination on whether or not the subject is able to perceive the orientation of the visual target 8 is made, for the color set in Step S1 is obtained.

When the above-mentioned threshold value is obtained for one visual target color, the threshold value for another visual target color is obtained by setting the combination of the visual target color and the background color to another combination (Steps S1 and S2 of FIG. 7) and repeating Steps S3 to S5. Finally, the above-mentioned threshold values for various visual target colors are obtained.

The examiner determines the presence or absence of acquired color vision deficiency, for example, based on the obtained threshold values for the visual target colors.

The effects of this embodiment will be described below. In this embodiment, the visual target 8 and the background 9 have virtually the same brightness (a brightness difference therebetween falls within plus or minus 10%), which makes it possible to properly test perception of the color of the visual target 8, not perception based on a brightness difference between the visual target 8 and the background 9.

Moreover, since the color of the visual target 8 and the color of the background 9 are set to a combination of colors which are in a relationship of confusion colors in dichromatism, a difference in test results (the above-mentioned threshold values) is more likely to be apparent between a person with an abnormality in cones and a person without an abnormality in cones or between a person with cones having low sensitivity and a person with cones having high sensitivity. In other words, it is possible to test cone-specific color visual acuity accurately. Specifically, for example, when the color of the visual target 8 and the color of the background 9 are set to a combination of confusion colors in protanopia, it is possible to accurately test the presence or absence of color vision deficiency related to L-cones and test whether the sensitivity of L-cones is high or low. Moreover, for example, when the color of the visual target 8 and the color of the background 9 are set to a combination of confusion colors in deuteranopia, it is possible to accurately test the presence or absence of color vision deficiency related to M-cones and test whether the sensitivity of M-cones is high or low. Furthermore, for example, when the color of the visual target 8 and the color of the background 9 are set to a combination of confusion colors in tritanopia, it is possible to accurately test the presence or absence of color vision deficiency related to S-cones and test whether the sensitivity of S-cones is high or low.

In addition, for example, the color of the visual target 8 and the color of the background 9 are set to chromatic colors (opposite colors) located on opposite sides of the achromatic region 110 on the confusion color loci 101 to 103, which allows a person without an abnormality in cones or a person with cones having high sensitivity to perceive the visual target 8 easily. As a result, a difference in test results (the above-mentioned threshold values) is more likely to be apparent between a person with an abnormality in cones and a person without an abnormality in cones or between a person with cones having low sensitivity and a person with cones having high sensitivity.

Moreover, for example, the color of the visual target 8 and the color of the background 9 are set to the same chroma, which makes it possible to test color visual acuity with the influence of the difference in chroma between the visual target 8 and the background 9 being suppressed.

Second Embodiment

Next, the second embodiment of this disclosure will be described with a focus mainly on a difference from the above-described embodiment. This embodiment is an embodiment in which the ability to see color in the peripheral vision is tested by presenting a visual target of a chromatic color at a position off the center of the visual field.

As in the case of the visual target display device of the first embodiment, a visual target display device of this embodiment has the configuration shown in FIG. 1. A visual target which is displayed by the display section 2 is different from that of the first embodiment. FIG. 8 illustrates display on the display section 2 of this embodiment. The display section 2 displays a reference point 21 for fixing a gaze of the subject thereon, a circular visual target 20 at a peripheral position of the reference point 21, and a background 22 around the reference point 21 and the visual target 20. The display section 2 displays the visual target 20 while changing the display position randomly among a plurality of predetermined measurement points 23. The display section 2 displays the visual target 20 at one measurement point 23 each time.

The measurement points 23 are set in a plurality of positions at different distances and in different directions from a center which is the position at which the reference point 21 is displayed. FIG. 8 shows an example in which the number of measurement points 23 is 16. These 16 measurement points 23 are set in such a way that they are arranged in four columns and four rows with the center located at the reference point 21. For example, the spacing between the measurement points 23 is determined in such a way that the visual angle between the adjacent measurement points 23 is a predetermined angle (for example, 1°) with the eyes of the subject being located at a predetermined distance (for example, 30 cm) from the screen of the display section 2.

Moreover, the display section 2 displays the visual target 20 at each measurement point 23 multiple times while changing the display position randomly among the plurality of measurement points 23. In doing so, the display section 2 does not display the visual target 20 successively at the same measurement point 23. That is, when the display section 2 displays the visual target 20 at one measurement point 23 multiple times, the display section 2 displays the visual target 20 at the one measurement point 23 and then displays the visual target 20 at another measurement point 23 or other measurement points 23 before displaying the visual target 20 at the one measurement point 23 again. Furthermore, the display section 2 changes the size of the visual target 20 when randomly (in other words, discontinuously) displaying the visual target 20 at each measurement point 23 multiple times. As in the case of the first embodiment, the background 22 is a plain image (without any pattern) of a single color.

The brightnesses and colors of the visual target 20 and the background 22 are set in the same manner as the first embodiment. That is, the visual target 20 and the background 22 are set to virtually the same brightness (so as to have a brightness difference falling within plus or minus 10%). Moreover, the color of the visual target 20 and the color of the background 22 are set to a combination of confusion colors in dichromatism.

The shape data 14 of the visual target data 12 (see FIGS. 1 and 2) which is stored in the storage section 4 is composed of data indicating a plurality of circles (visual targets 20) having different diameters. Moreover, the color data 15 of the visual target data 12 is configured in the same manner as the first embodiment.

Furthermore, the content of the program 13 which is stored in the storage section 4 is different from that of the first embodiment. Specifically, the program 13 is different from that of the first embodiment in that the program 13 causes the control section 3 (a computer) to function as display control means that causes the display section 2 to display the visual target 20 while changing the size and display position thereof.

The response input section 7 of FIG. 1 is configured as an operation section which is operated by the subject, and is a section by which the subject performs input of a signal indicating that the subject has been able to perceive the visual target 20. The response input section 7 includes, for example, a holding section that is held by the subject and an operation section on which a pressing operation is performed by the thumb of the subject holding the holding section.

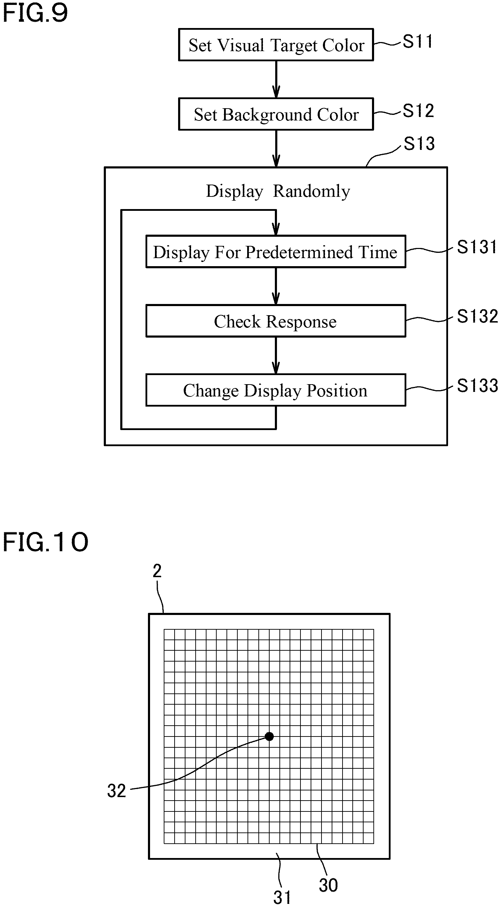

Next, a procedure of a method of testing the ability to see color of this embodiment will be described with reference to FIG. 9. A test is carried out in a dark room to eliminate the influence of extraneous light. The subject is positioned at a predetermined distance from the screen of the display section 2. An examiner starts the program 13 (see FIG. 1) and causes the control section 3 to execute the program 13. When the program 13 is started, the control section 3 causes the auxiliary display section 6 to display the screen for specifying the visual target color and the background color. For example, combinations (A1, B1), (A2, B2), (A3, B3), ... of the visual target color and the background color indicated by the color data 15 of FIG. 2 are displayed on this screen.

First, as in the case of the first embodiment, the control section 3 sets the visual target color and the background color, which are in a relationship of confusion colors, based on an input signal from the operation section 5 (Steps S11 and S12 of FIG. 9).

Then, the control section 3 causes the display section 2 to display the reference point 21 (see FIG. 8) and gets the subject to fix his/her gaze at the reference point 21. The control section 3 then reads the data A on the visual target color set in Step S11 and the data B on the background color set in Step S12 from the storage section 4 and causes the display section 2 to display the visual target 20 while changing the display position randomly among the plurality of predetermined measurement points 23 with the read visual target color A and background color B being maintained (Step S13 of FIG. 9).

Specifically, in Step S13, the control section 3 causes the display section 2 to display the visual target 20 at one of the plurality of measurement points 23 for a predetermined time (Step S131), checks whether or not a response indicating that the subject was able to perceive the visual target 20 has been made from the response input section 7 in the predetermined time (Step S132), and changes the display position of the visual target 20 to another measurement point 23 after a lapse of the predetermined time (Step S133). The control section 3 causes the display section 2 to discontinuously display the visual target 20 at each measurement point 23 multiple times by repeating Steps S131 to S133.

Moreover, when the control section 3 causes the display section 2 to display the visual target 20 at each measurement point 23 multiple times while changing the display position randomly, if a signal indicating that the subject was able to perceive the visual target 20 is input for the last display from the response input section 7, the control section 3 sets the size of the visual target 20 which is displayed this time to a smaller value than that of the last time. Furthermore, if a signal is not input for the last display from the response input section 7, the control section 3 sets the size of the visual target 20 which is displayed this time to a larger value than that of the last time. Finally, a threshold value, which is the size of the visual target 20 based on which a determination on whether or not the subject is able to perceive the visual target 20 is made, is obtained for each measurement point 23.

When the threshold value for each measurement point 23 is obtained for one visual target color, the above-mentioned threshold value for each measurement point 23 is also obtained for another visual target color by setting the combination of the visual target color and the background color to another combination (Steps S11 and S12 of FIG. 9) and executing Step S13. Finally, the above-mentioned threshold value for each measurement point 23 is obtained for each of various visual target colors.

As described above, in this embodiment, in addition to being able to obtain the effects similar to those of the first embodiment, it is possible to accurately test the ability (color visual acuity) to distinguish a color at each measurement point 23 in the visual field. In other words, it is possible to test the visual field of color accurately.

Third Embodiment

Next, a third embodiment of this disclosure will be described with a focus mainly on a difference from the above-described embodiments. This embodiment is an embodiment in which the visual field of color is tested by presenting a visual target of a chromatic color simultaneously in a large area of the visual field and getting the subject to make a response about the presence or absence of a region (a defective region or a distorted region) where the subject is not able to perceive the visual target properly. As in the case of the visual target display devices of the first and second embodiments, a visual target display device of this embodiment has the configuration shown in FIG. 1. A visual target which is displayed by the display section 2 is different from those of the first and second embodiments. FIG. 10 illustrates display on the display section 2 of this embodiment.

The display section 2 displays a grid 30 (a lattice), which resembles the Amsler chart in shape, as a visual target and a background 31 around the grid 30 (in a region other than the grid 30). Unlike the conventional Amsler chart, the color of the grid 30 and the color of the background 31 are both chromatic colors. Specifically, as in the case of the first and second embodiments, the color of the grid 30 and the color of the background 31 are set to a combination of colors which are in a relationship of confusion colors in dichromatism. Moreover, the brightness of the grid 30 and the brightness of the background 31 are set to virtually the same brightness (so as to have a brightness difference falling within plus or minus 10%). As described above, the brightnesses and colors of the visual target 30 (the grid) and the background 31 are set in the same manner as the first embodiment.

The grid 30 has a shape in which, when lines forming a square are defined as a minimum constituent unit, the minimum constituent units are continuously arranged in a plurality of columns and rows. The minimum constituent units (the lines forming the squares) are set to the same shape and the same color. Moreover, the region inside each minimum constituent unit (the lines forming a square) is set to the background 31. The size of each minimum constituent unit is not limited, and for example, the size of each minimum constituent unit is set to a size that attains a 1-degree visual angle between the adjacent horizontal lines and between the adjacent vertical lines with the eyes of the subject being located at a predetermined distance (for example, about 30 cm) from the grid 30.

The background 31 is a plain image (without any pattern) of a single color.

The shape data 14 of the visual target data 12 (see FIGS. 1 and 2) which is stored in the storage section 4 is the shape data on the grid 30 shown in FIG. 10. Moreover, the color data 15 of the visual target data 12 is configured in the same manner as the first embodiment.

Furthermore, the content of the program 13 which is stored in the storage section 4 is different from that of the first embodiment. Specifically, the program 13 is different from that of the first embodiment in that the program 13 causes the control section 3 (a computer) to function as display control means that causes the display section 2 to display the grid 30 and the background 31 of FIG. 10.

In this embodiment, the response input section 7 of FIG. 1 is not used. Therefore, the visual target display device 1 of this embodiment does not necessarily have to include the response input section 7.

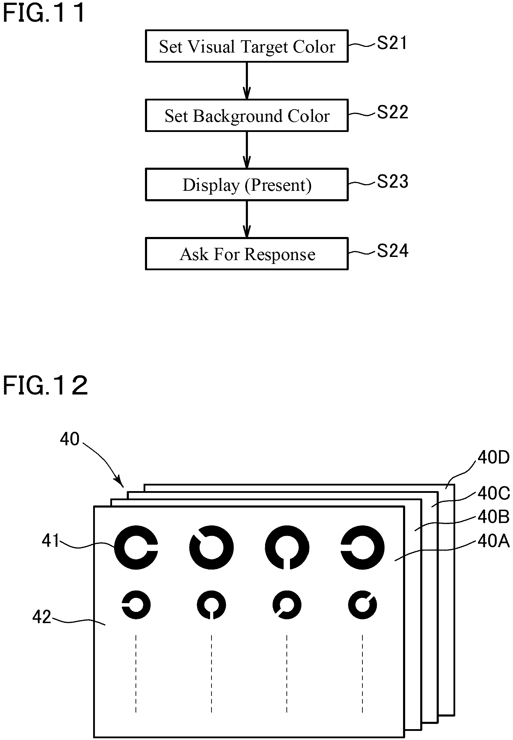

Next, a procedure of a method of testing the ability to see color of this embodiment will be described with reference to FIG. 11. A test is carried out in a dark room to eliminate the influence of extraneous light. The subject is positioned at a predetermined distance from the screen of the display section 2. An examiner starts the program 13 (see FIG. 1) and causes the control section 3 to execute the program 13. When the program 13 is started, the control section 3 causes the auxiliary display section 6 to display the screen for specifying the visual target color and the background color. For example, combinations (A1, B1), (A2, B2), (A3, B3), ... of the visual target color and the background color indicated by the color data 15 of FIG. 2 are displayed on this screen.

First, as in the case of the first embodiment, the control section 3 sets the visual target color and the background color, which are in a relationship of confusion colors, based on an input signal from the operation section 5 (Steps S21 and S22 of FIG. 11).

Then, the control section 3 reads the data A on the visual target color set in Step S21 and the data B on the background color set in Step S22 from the storage section 4, causes the display section 2 to display the grid 30 of the read visual target color A and the background 31 of the read background color B, and presents the grid 30 to the subject (Step S23 of FIG. 11).

Then, the subject is asked to look at a center 32 of the grid 30 and, in this state, make a verbal response as to the presence or absence of a region which the subject is not able to see in the grid 30 or a region which looks distorted in the grid 30 (Step S24 of FIG. 11). If there is a region which the subject is not able to see in the grid 30 or a region which looks distorted in the grid 30, the subject is asked to describe the region specifically.

When a response in Step S24 to one visual target color is obtained, the above-mentioned response to another visual target color is also obtained by setting the combination of the visual target color and the background color to another combination (Steps S21 and S22 FIG. 11) and executing Steps S23 and S24. Finally, the above-mentioned responses to various visual target colors are obtained.

As described above, in this embodiment, in addition to being able to obtain the effects similar to those of the first embodiment, it is possible to identify an abnormal perception region in the visual field on a color-by-color basis in a concrete manner and test the visual field of color of the subject in the form of, for example, a finding of an abnormality in an area on a retina which corresponds to that region.

Modifications

This disclosure is not limited to the above-described embodiments and can be modified in various ways. Modifications will be described below. The above-described embodiments deal with an example in which a visual target and a background are displayed on a display section that electrically displays an image. However, a visual target and a background may be printed on paper, resin, or the like. FIG. 12 shows a modification of the first embodiment, illustrating a sheet 40 with a visual target and a background printed thereon as a visual target display device. The sheet 40 is formed of paper, resin (plastic), metal, or the like. On the surface of the sheet 40, a plurality of Landolt rings 41 which have various sizes and orientations are printed as visual targets and a background 42 is printed around the Landolt rings 41. The surface of the sheet 40 serves as a display section that displays the visual targets 41 and the background 42.

The visual targets 41 which are displayed on one sheet 40 are set to the same chromatic color. Moreover, the background 42 is set to a plain background (without any pattern) of a single chromatic color. As in the case of the first to third embodiments, the brightness of the visual target 41 and the brightness of the background 42 are set to virtually the same brightness (so as to have a brightness difference falling within plus or minus 10%). Furthermore, as in the case of the first to third embodiments, the color of the visual target 41 and the color of the background 42 are set to a combination of colors which are in a relationship of confusion colors in dichromatism.

The sheet 40 includes a plurality of sheets 40A, 40B, 40C, 40D, ... which are different in a combination of the color of the visual target 41 and the color of the background 42. One of the plurality of sheets 40A, 40B, 40C, 40D, ... displays the visual target 41 and the background 42 which are in a relationship of confusion colors in protanopia, for example. Another sheet of the plurality of sheets 40A, 40B, 40C, 40D, ... displays the visual target 41 and the background 42 which are in a relationship of confusion colors in deuteranopia, for example. Still another sheet of the plurality of sheets 40A, 40B, 40C, 40D, ... displays the visual target 41 and the background 42 which are in a relationship of confusion colors in tritanopia, for example.

A procedure for testing the ability to see color using the sheet 40 is as follows. An examiner selects one of the plurality of sheets 40 (40A, 40B, 40C, 40D, ...). Then, the examiner presents the selected sheet 40 to the subject, and indicates one of the plurality of visual targets 41 displayed on the sheet 40 with a stick or the like and asks the subject to make a response as to whether he/she is able to perceive the orientation of the visual target 41. The size or orientation of the visual target 41 that is indicated by the stick or the like is then changed to another size or orientation and, finally, a threshold value, which is the size of the visual target 41 based on which a determination on whether or not the subject is able to perceive the visual target 41 is made, is obtained.

When the above-mentioned threshold value is obtained for one visual target color, the sheet 40 of another visual target color is selected and the above-mentioned threshold value is also obtained for that visual target color. Finally, the above-mentioned threshold values for various visual target colors are obtained. This also makes it possible to obtain the effects similar to those of the first embodiment.

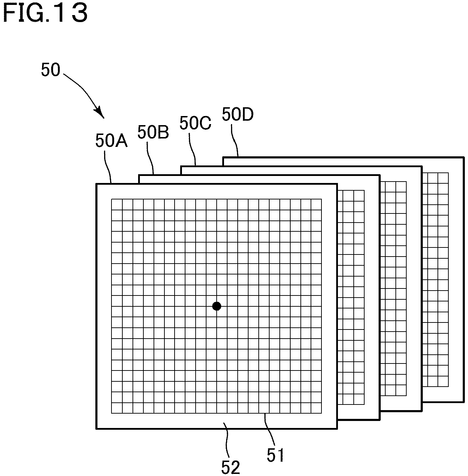

FIG. 13 shows a modification of the third embodiment, illustrating a sheet 50 with a visual target and a background printed thereon as a visual target display device. The sheet 50 is formed of paper, resin (plastic), metal, or the like. On the surface of the sheet 50, a grid 51 similar to that of the third embodiment is printed and a background 52 is printed around the grid 51. The brightnesses and colors of the visual target 51 and the background 52 are set in the same manner as the third embodiment.

The sheet 50 includes a plurality of sheets 50A, 50B, 50C, 50D, ... which are different in a combination of the color of the visual target 51 and the color of the background 52. One of the plurality of sheets 50A, 50B, 50C, 50D, ... displays the visual target 51 and the background 52 which are in a relationship of confusion colors in protanopia, for example. Another sheet of the plurality of sheets 50A, 50B, 50C, 50D, ... displays the visual target 51 and the background 52 which are in a relationship of confusion colors in deuteranopia, for example. Still another sheet of the plurality of sheets 50A, 50B, 50C, 50D, ... displays the visual target 51 and the background 52 which are in a relationship of confusion colors in tritanopia, for example.

A procedure for testing the ability to see color using the sheet 50 is as follows. An examiner selects one of the plurality of sheets 50 (50A, 50B, 50C, 50D, ...). Then, the examiner presents the selected sheet 50 to the subject and tests whether or not there is a region which the subject is not able to see in the grid 51 or a region which looks distorted in the grid 51. After carrying out the test on one visual target color, the examiner selects the sheet 50 of another visual target color and carries out the test on that visual target color to find out whether or not there is an abnormal perception region in the grid 51. This test is carried out on various visual target colors. This also makes it possible to obtain the effects similar to those of the third embodiment.

As an example of the visual target, the above-described embodiments illustrate a Landolt ring (the first embodiment), a circular visual target (the second embodiment), and a grid in which squares are arranged in columns and rows (the third embodiment). However, the visual target may have any shape. For example, in the second embodiment, the visual target may be a polygon (a quadrangle), an ellipse, or the like, or may be a pattern, a figure (such as an animal, a plant, and food), or the like. Moreover, in the third embodiment, a shape configured with dots, circles, polygons (quadrangles), ellipses, patterns, figures (such as animals, plants, and food), or the like arranged in columns and rows may be adopted as the visual target.

Furthermore, the above-described embodiments illustrate an example in which the visual target color and the background color are set to chromatic colors (opposite colors) located on opposite sides of a region of an achromatic color on a confusion color locus. However, the visual target color and the background color do not necessarily have to be set to opposite colors. Specifically, for example, the visual target color and the background color may be set to chromatic colors in the same region out of two chromatic regions on a confusion color locus which are sectioned with an achromatic region located therebetween. This makes a color difference between the visual target color and the background color small compared to when the visual target color and the background color are set to opposite colors and thereby makes it possible to determine the sensitivity of cones more finely. That is, a fact that the subject can distinguish a smaller color difference between the visual target color and the background color means that the sensitivity of cones is higher.

Moreover, the above-described embodiments illustrate an example in which the visual target color and the background color are set to colors on a confusion color locus passing through an achromatic region. However, the visual target color and the background color may be set to colors (including colors similar to an on-locus color) on a confusion color locus that does not pass through an achromatic region.

In addition, the above-described embodiments illustrate an example in which the visual target color and the background color are set to colors of the same chroma. However, the visual target color and the background color may be set to colors of different chromas.

Moreover, the above-described embodiments illustrate an example in which the background is set to a chromatic color. However, the background may be set to an achromatic color as long as the visual target color and the background color are in a relationship of confusion colors.

Furthermore, a configuration in which one or both of the visual target color and the background color can be set to any color on a confusion color locus may be adopted. In this case, for example, an xy chromaticity diagram and a confusion color locus may be displayed on the auxiliary display section 6 of FIG. 1 to allow an examiner to specify any point on the confusion color locus using the operation section 5 or the like and thereby present a visual target or background of a color corresponding to the specified point to the subject.

DESCRIPTION OF THE REFERENCE CHARACTERS

1, 40, 50 visual target display device
2, 40, 50 display section
3 control section
8, 20, 30, 41, 51 visual target
9, 22, 31, 42, 52 background
101 confusion color locus in protanopia
102 confusion color locus in deuteranopia
103 confusion color locus in tritanopia

**Claims**

1.  A visual target display device (1, 40) comprising

    a display section (2, 40) configured to display a visual target (8, 20, 41), of a chromatic color, for testing a color-related visual function of a subject and a background (9, 22, 42) around the visual target (8, 20, 41), wherein a brightness of the background (9, 22, 42) is a numerical value between a numerical value 10% less than a brightness of the visual target (8, 20, 41) and a numerical value 10% greater than the brightness of the visual target (8, 20, 41),
    a combination of a color of the visual target (8, 20, 41) and a color of the background (9, 22, 42) is a combination of colors which are in a relationship of confusion colors in dichromatism, and
    the display section (2, 40) changes a size of the visual target (8, 20) or displays a plurality of the visual targets (41) of different sizes while maintaining the combination of the color of the visual target (8, 20, 41) and the color of the background (9, 22, 42), so as to obtain a threshold value which is a size of the visual target (8, 20, 41) based on which a determination on whether or not the subject is able to perceive the visual target (8, 20, 41) is made.

2.  The visual target display device (1, 40) according to claim 1, wherein

    the visual target (8, 41) is a visual target which is presented in a center of a visual field of the subject,
    the threshold value is a size of the visual target (8, 41) based on which a determination on whether or not the subject is able to perceive an orientation of the visual target (8, 41) is made when the visual target (8, 41) is presented in the center of the visual field of the subject, and
    the display section (2 ,40) changes a size and an orientation of the visual target (8) or displays a plurality of the visual targets (41) of different sizes and orientations while maintaining the combination of the color of the visual target (8, 41) and the color of the background (9, 42), so as to obtain the threshold value.

3.  The visual target display device (1) according to claim 1, wherein
    the display section (2) displays the visual target (20) while changing a display position randomly among a plurality of predetermined measurement points (23) each at a peripheral position off a center (21) of a visual field of the subject and changing a size of the visual target (20) with the combination of the color of the visual target (20) and the color of the background (22) being maintained.

4.  A visual target display device (1, 50) comprising

    a display section (2, 50) configured to display a visual target (30, 51), of a chromatic color, for testing a color-related visual function of a subject and a background (31, 52) around the visual target (30, 51), wherein a brightness of the background (31, 52) is a numerical value between a numerical value 10% less than a brightness of the visual target (30, 51) and a numerical value 10% greater than the brightness of the visual target (30, 51),
    a combination of a color of the visual target (30, 51) and a color of the background (31, 52) is a combination of colors which are in a relationship of confusion colors in dichromatism,

the visual target (30, 51) has a shape in which a plurality of minimum constituent units set to a same shape and a same color are arranged in columns and rows, and

the visual target (30, 51) is a visual target for testing whether or not there is a region which the subject is not able to see in the visual target (30, 51) or a region which looks distorted in the visual target (30, 51) while the subject is looking at a center (32) of the visual target (30, 51).

5. The visual target display device (1, 40, 50) according to any one of claims 1 to 4, wherein
the color of the background (9, 22, 31, 42, 52) is a chromatic color.

6. The visual target display device (1, 40, 50) according to any one of claims 1 to 5, wherein
the color of the visual target (8, 20, 30, 41, 51) and the color of the background (9, 22, 31, 42, 52) are set to a combination of chromatic colors which are in the relationship of confusion colors located on opposite sides of a region (110) of an achromatic color in a chromaticity diagram.

7. The visual target display device (1, 40, 50) according to any one of claims 1 to 5, wherein
the color of the visual target (8, 20, 30, 41, 51) and the color of the background (9, 22, 31, 42, 52) are set to a combination of chromatic colors which are in the relationship of confusion colors in a same region out of two chromatic regions which are sectioned with a region (110) of an achromatic color in a chromaticity diagram located therebetween.

8. The visual target display device (1, 40, 50) according to any one of claims 1 to 7, wherein
the color of the visual target (8, 20, 30, 41, 51) and the color of the background (9, 22, 31, 42, 52) are set to a same chroma.

9. The visual target display device (1, 40, 50) according to any one of claims 1 to 7, wherein
the color of the visual target (8, 20, 30, 41, 51) and the color of the background (9, 22, 31, 42, 52) are set to different chromas.

10. A visual target presentation method comprising:

presenting a visual target (8, 20, 41), of a chromatic color, for testing a color-related visual function of a subject and a background (9, 22, 42) around the visual target (8, 20, 41) in such a way that a brightness of the background (9, 22, 42) is a numerical value between a numerical value 10% less than a brightness of the visual target (8, 20, 41) and a numerical value 10% greater than the brightness of the visual target (8, 20, 41) and a combination of a color of the visual target (8, 20, 41) and a color of the background (9, 22, 42) is a combination of colors which are in a relationship of confusion colors in dichromatism; and

obtaining a threshold value, which is a size of the visual target (8, 20, 41) based on which a determination on whether or not the subject is able to perceive the visual target (8, 20, 41) is made, by changing a size of the visual target (8, 20, 41) presented to the subject while maintaining the combination of the color of the visual target (8, 20, 41) and the color of the background (9, 22, 42).

11. A program (13) for causing a computer (3) to function as display control means configured to cause a display section (2) to display a visual target (8, 20), of a chromatic color, for testing a color-related visual function of a subject and a background (9, 22) around the visual target (8, 20) in such a way that a brightness of the background (9, 22) is a numerical value between a numerical value 10% less than a brightness of the visual target (8, 20) and a numerical value 10% greater than the brightness of the visual target (8, 20) and a combination of a color of the visual target (8, 20) and a color of the background (9, 22) is a combination of colors which are in a relationship of confusion colors in dichromatism, wherein

the display control means (3) changes a size of the visual target (8, 20) while maintaining the combination of the color of the visual target (8, 20) and the color of the background (9, 22), so as to obtain a threshold value which is a size of the visual target (8, 20) based on which a determination on whether or not the subject is able to perceive the visual target (8, 20) is made.

# FIG.1

Control Section

10
Visual Target Control Section

11
Background Control Section

Storage Section

Visual Target Data — 12

Program — 13

Operation Section — 5

Auxiliary Display Section — 6

Response Input Section — 7

# FIG.2

12

Visual Target Data

Shape Data

14

| Color Data | |
| Visual Target Color A | Background Color B |
| --- | --- |
| A1 | B1 |
| A2 | B2 |
| A3 | B3 |
| ⋮ | ⋮ |

15

# FIG.3

FIG.4

# FIG.5

# FIG.6

| | |
|---|---|
| – – – – | Tritanopia Confusion Color Locus |
| ---■--- | Chroma 8 |
| ---●--- | Chroma 6 |
| ---■--- | Chroma 4 |
| ---●--- | Chroma 2 |
| ✕ | D65 |

FIG.7

```
          ┌─────────────────────────┐
          │  Set Visual Target Color│ ─S1
          └─────────────────────────┘
                      │
                      ▼
          ┌─────────────────────────┐
          │   Set Background Color   │ ─S2
          └─────────────────────────┘
                      │
                      ▼
   ┌──────────────────────────────────────────┐
   │ Set Shape (Size, Orientation) Of Visual Target│ ─S3
   └──────────────────────────────────────────┘
                      │
                      ▼
          ┌─────────────────────────┐
          │     Display (Present)    │ ─S4
          └─────────────────────────┘
                      │
                      ▼
          ┌─────────────────────────┐
          │     Ask For Response     │ ─S5
          └─────────────────────────┘
```

FIG.8

## FIG.9

Set Visual Target Color — S11

Set Background Color — S12

S13
Display Randomly

Display For Predetermined Time — S131

Check Response — S132

Change Display Position — S133

## FIG.10

2

32

31  30

FIG.11

```
Set Visual Target Color ──S21
          │
          ▼
Set Background Color ──S22
          │
          ▼
Display (Present) ──S23
          │
          ▼
Ask For Response ──S24
```

FIG.12

FIG.13

FIG.14

Europäisches
Patentamt

European
Patent Office

Office européen
des brevets

# EUROPEAN SEARCH REPORT

Application Number

EP 22 18 9664

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 417 764 B1 (CHUKYO MEDICAL CO INC [JP]) 30 October 2019 (2019-10-30) <br> * paragraph [0002] * <br> * paragraph [0006] * <br> * paragraph [0016] * <br> * paragraph [0020] * <br> * paragraph [0022] - paragraph [0025] * <br> * paragraph [0033] - paragraph [0034] * <br> * paragraph [0056] * <br> * paragraph [0063] * <br> ----- | 1-11 | INV. <br> A61B3/06 |
| Y | US 9 826 898 B1 (JIN CAN [US] ET AL) 28 November 2017 (2017-11-28) <br> * column 5, line 66 - column 6, line 27 * <br> * column 7, line 30 - line 61 * <br> * column 9, line 35 - column 10, line 13 * <br> * column 10, line 39 - column 11, line 8 * <br> ----- | 1-11 | |
| Y | EP 2 839 771 A1 (ICHIKAWA KAZUO [JP]; UNIV SHINSHU [JP]; KOWA CO [JP]) 25 February 2015 (2015-02-25) <br> * paragraph [0004] * <br> * paragraph [0007] * <br> * paragraph [0029] - paragraph [0031] * <br> * figures 2,4 * <br> ----- | 1-11 | TECHNICAL FIELDS SEARCHED (IPC) <br><br> A61B |

The present search report has been drawn up for all claims

| Place of search | Date of completion of the search | Examiner |
|---|---|---|
| Munich | 14 December 2022 | Horváth, László |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 18 9664

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2022

| Patent document cited in search report | | Publication date | Patent family member(s) | | Publication date |
|---|---|---|---|---|---|
| EP 3417764 | B1 | 30-10-2019 | CN | 109008939 A | 18-12-2018 |
| | | | EP | 3417764 A1 | 26-12-2018 |
| | | | JP | 6184046 B1 | 23-08-2017 |
| | | | JP | 2018202090 A | 27-12-2018 |
| | | | US | 2019133439 A1 | 09-05-2019 |
| US 9826898 | B1 | 28-11-2017 | NONE | | |
| EP 2839771 | A1 | 25-02-2015 | EP | 2839771 A1 | 25-02-2015 |
| | | | JP | 6212034 B2 | 11-10-2017 |
| | | | JP | WO2013157573 A1 | 21-12-2015 |
| | | | US | 2015085258 A1 | 26-03-2015 |
| | | | WO | 2013157573 A1 | 24-10-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5436712 B
- JP 6184046 B